# EUROPEAN PATENT APPLICATION

(11) **EP 3 421 484 A1**
(43) Date of publication of application: **02.01.2019**
(21) Application number: 17755845.9
(22) Date of filing: 24.02.2017
(51) Int. Cl.: C07K 7/08, C07K 1/113, A61K 38/10, A61P 11/00

(54) **POLYPEPTIDE, DERIVATIVES THEREOF, AND APPLICATION THEREOF IN PREPARATION OF DRUGS HAVING RESISTANCE TO PULMONARY FIBROSIS**

(30) Priority: 26.02.2016 CN 201610108248
(71) Applicant: Hu, Zhuowei, Beijing 100102 (CN)
(72) Inventor: LV, Xiaoxi, Beijing 100102 (CN); LIU, Shanshan, Beijing 100102 (CN); HU, Zhuowei, Beijing 100102 (CN)
(74) Representative: dompatent von Kreisler Selting Werner - Partnerschaft von Patent- und Rechtsanwälten mbB
(86) International application number: PCT/CN2017/074792
(87) International publication number: WO 2017/144016

(57) **Abstract**

Provided are a polypeptide specifically binding to TRB3 and application thereof in preparation of drugs for treating and/preventing pulmonary fibrosis. The amino acid sequence of the polypeptide is represented by SEQ ID No. 12 in a sequence list, or two or more amino acids in the amino acid sequence as represented by SEQ ID No. 12 in the sequence list are replaced with unnatural amino acids having side chains that can be linked to each other. The polypeptide and derivatives thereof can specifically bind to TRB3, thereby blocking the interaction between TRB3 and MDM2 proteins.

## Description

The present application claims priority of Chinese Patent Application No. CN201610108248.9, filed on Feb. 26, 2016. The entire content of the aforementioned application is hereby incorporated by reference.

### Field of the Invention

The invention relates to the field of biotechnology, in particular relates to a polypeptide, derivatives thereof, and application thereof in preparation of drugs having resistance to pulmonary fibrosis.

### Background of the Invention

Pulmonary fibrosis is one of the most serious pathological conditions in lungs. Most of the pathological changes are initial inflammation of the lower respiratory tract, as well as damages of alveolar epithelial cells and vascular endothelial cells, accompanied by proliferation of fibroblasts and alveolar type II cells and cytokines release, and the deposition of extracellular matrix protein and collagen, which finally cause changes in lungs. The pulmonary alveoli in patients with pulmonary fibrosis are gradually replaced by fibrotic substances, resulting in hardening and thickening of lung tissues, and gradual loss of gas exchange capacity in lung, which further causes dyspnea in patients owing to various degrees of hypoxia, and finally death due to respiratory failure. Having a complicated etiology and unknown pathogenesis, pulmonary fibrosis is one of the four major entities of respiratory diseases. The existing medicaments and methods for treating pulmonary fibrosis are very limited, meanwhile, the curative effect is not satisfactory and the prognosis is very poor with only 50% of 5-year survival rate.

TRB3 (Tribbles Homologue 3), one member of the pseudokinase Tribbles homologous protein family in human body, has protein linker-like functions and participates in the assembly of various protein complexes. A number of studies indicate that TRB3 can interact with a variety of transcription factors, ubiquitin ligase, cell membrane receptors and MAPK and PI3K signaling pathway member proteins, and participate in cell differentiation, apoptosis, autophagy and metabolic regulation. Recently, a variety of evidences suggest that TRB3 is highly expressed in pulmonary fibrosis and plays a significant role in promoting the development of pulmonary fibrosis. MDM2 is the E3 ubiquitin ligase in the ubiquitin proteasome system.

### Summary of the Invention

The technical problem to be solved by the present invention is to provide a polypeptide, derivatives thereof and the application thereof in preparation of drugs for treating and/or preventing pulmonary fibrosis in view of the unavailability of a safe and effective medicament against pulmonary fibrosis. The polypeptide targets the interaction between TRB3 and MDM2, and thus can be used in the preparation of a medicament for preventing and/or treating pulmonary fibrosis with high activity and selectivity.

Upon intensive studies and repeated experiments, the inventors of this invention found that TRB3 inhibits the degradation of SLUG protein that promotes epithelial-mesenchymal transition through the interaction with MDM2, thereby promoting pulmonary fibrosis. Therefore, the interaction between TRB3 and MDM2 is a potential target for the prevention and/or treatment of pulmonary fibrosis. In another word, blocking the interaction between TRB3 and MDM2 is a potential way for the prevention and/or treatment of pulmonary fibrosis, and there is a need for developing a drug that can block the mentioned interaction. The inventors obtained a polypeptide MR2 (the amino acid sequence of which is shown in SEQ ID No. 12 of the Sequence Listing) that targets TRB3 and interacts with MDM2 protein. Meanwhile, the inventors found that if the amino acid residue at a specific position in the polypeptide MR2 is substituted with a unnatural amino acid with side chains that can be linked, such as S-Pentenyl alanine (S5), the resulting modified polypeptide will possess a stable secondary structure of alpha helix, thus having extremely high affinity, anti-enzymatic stability and cell membrane permeability. Therefore, the engineered polypeptide having high alpha helix stability, TRB3 binding ability and metabolic stability can be used for the preparation of a medicament for treating and/or preventing pulmonary fibrosis. Based on the research work of the inventors, the present invention provides the following technical solutions.

In a first aspect, the present invention provides a polypeptide or derivatives thereof that specifically binds to TRB3, wherein the amino acid sequence of the polypeptide is represented by SEQ ID No. 12 in a Sequence List, or two or more amino acids in the amino sequence as represented by SEQ ID No. 12 in the Sequence List are replaced by unnatural amino acids having side chains that can be linked. The derivatives include the chimeric peptide formed by linking the polypeptide to a cell membrane penetrating peptide, the fusion peptide formed by the polypeptide and virus, the polypeptide that is methylated, the polypeptide that is glycosylated, and the polypeptide that is PEGylated.

The amino acid sequence of polypeptide setting forth in SEQ ID NO. 12 is referred to as MR2 polypeptide.

The unnatural amino acid with side chain that can be linked is conventional unnatural amino acid in the art, preferably S-Pentenyl alanine (S5).

Among them, the cell membrane penetrating peptide of present invention is conventional cell membrane penetrating peptide in the art as long as it can assist in delivering the polypeptide into the cell to play its role. Generally, the cell membrane penetrating peptide is a short peptide molecule composed of 10-30 amino acids. Preferably, the cell membrane penetrating peptide is linked to N terminal or C terminal of the polypeptide, more preferably to N terminal of the polypeptide. Preferably, the cell membrane penetrating peptide and the polypeptide or polypeptide derivatives that specifically binds to TRB3 are linked by two glycines (Gly-Gly).

Preferably, the cell membrane penetrating peptide is one or more peptides selected from HIV-1 virus trans-activator transcription (TAT) TAT peptide (amino acid sequence of which is shown in SEQ ID No. 13 of the Sequence Listing), the transcription factor Antp peptide of *Drosophila* antennal homeotic protein (amino acid sequence of which is shown in SEQ ID No. 14 of the Sequence Listing), Pep-1 peptide (amino acid sequence of which is shown in SEQ ID No. 15 of the Sequence Listing), MPG peptide (amino acid sequence of which is shown in SEQ ID No. 16 of the Sequence Listing) and RGD peptide (amino acid sequence of which is shown in SEQ ID No. 17 of the Sequence Listing). More preferably, the cell membrane penetrating peptide is Pep peptide (amino acid sequence of which is shown in SEQ ID No. 18 of the Sequence Listing).

Preferably, the chimeric peptide is a chimeric polypeptide Pep2-MR2 (amino acid sequence of which is shown in SEQ ID No. 19 of the Sequence Listing) formed by linking the pep2 peptide to the MR2 polypeptide, a chimeric polypeptide TAT-MR2 formed by linking TAT polypeptide to MR2 polypeptide (amino acid sequence of which is shown in SEQ ID No. 20 of the Sequence Listing), or a chimeric polypeptide Antp-MR2 formed by connecting Antp peptide with MR2 polypeptide (amino acid sequence of which is shown in SEQ ID No. 21 of the Sequence Listing).

Preferably, the unnatural amino acid having side chain that can be linked is S-Pentenyl alanine. In the polypeptide, the number of the replaced amino acids is two and the positions of the replaced amino acids are at position of i^{th} and (i+3)^{th}, or at position of i^{th} and (i+4)^{th} respectively, wherein the "i" is a positive integer ranges from 1 to 11 (1 ≤i≤11). Among them, both "i+3" and "i+4" increase the helix ratio of the polypeptide, thereby improving the stability of the polypeptide.

More preferably, the amino acid sequence of the polypeptide is shown in any one of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, and SEQ ID NO. 11 of the Sequence Listing.

Among them, the amino acid sequences of SEQ ID No. 1-11 can be appropriately substituted, deleted or added as long as the engineered amino acid sequence can still specifically bind to TRB3 and maintain the activity before engineering.

In a second aspect, the present invention provides an application of the polypeptide or the derivatives thereof that specifically binds to TRB3 in the preparation of drugs for treating and/or preventing pulmonary fibrosis.

The "Pulmonary fibrosis" described herein is a conventional pulmonary fibrosis in the art. It preferably refers to pulmonary fibrosis characterized by pathological changes of idiopathic pulmonary fibrosis caused by various factors. Among them, the pulmonary fibrosis preferably refers to pulmonary fibrosis of a human or an animal. More preferably, the symptoms of the pulmonary fibrosis include pulmonary inflammation and pulmonary function degeneration caused by pulmonary fibrosis. Preferably, the causes of the pulmonary fibrosis comprise lung injury, dust or drugs.

The drug described herein is a conventional drug in the art which can induce pulmonary fibrosis, preferably bleomycin.

Among them, the pulmonary fibrosis is preferably primary (idiopathic) pulmonary fibrosis, i.e. agnogenic pulmonary fibrosis; or preferably secondary pulmonary fibrosis, i.e. pulmonary fibrosis secondary to original disease; more preferably, pulmonary function degeneration, lung inflammation and lung injury in pulmonary fibrosis. Diseases of pulmonary fibrosis preferably includes chronic obstructive pulmonary disease (COPD), idiopathic pulmonary fibrosis or interstitial pneumonia.

Terms "Preventing" or "Prevention" used herein means preventing or reducing the occurrence of pulmonary fibrosis after administration in the presence of possible pulmonary fibrosis factors. "Treating" or "Treatment" used herein means reducing the severity of pulmonary fibrosis, either curing pulmonary fibrosis, or slowing down the progress of pulmonary fibrosis.

In a third aspect, the present invention provides a pharmaceutical composition having resistance to pulmonary fibrosis, comprising the polypeptide or derivatives thereof that specifically binds to TRB3 as an active ingredient.

The "active ingredient" refers to a compound possessing the function of preventing or treating pulmonary fibrosis. In the pharmaceutical composition, the polypeptide or derivatives thereof that specifically binds to TRB3 acts as active ingredients alone or together with other compounds having anti-pulmonary fibrosis activity.

The administration route of the pharmaceutical composition in the present invention is preferably administered by injection or administered orally. The injection administration preferably includes intravenous injection, intramuscular injection, intraperitoneal injection, intradermal injection or subcutaneous injection, etc. The pharmaceutical composition is a variety of dosage forms conventionally known in the art, preferably in the form of solid, semisolid or liquid such as aqueous solutions, nonaqueous solutions or suspensions, and more preferably tablets, capsules, granules, injections or infusions and the like.

Preferably, the pharmaceutical composition of the present invention further comprises one or more pharmaceutical carriers. The pharmaceutical carrier is the conventional pharmaceutical carrier known in the art, and the pharmaceutical carrier can be any suitable physiologically or pharmaceutically acceptable pharmaceutical adjuvant. The pharmaceutical adjuvant is the conventional pharmaceutical adjuvant known in the art, preferably pharmaceutically acceptable excipients, fillers, diluents and the like. More preferably, the pharmaceutical composition comprises 0.01-99.99% of aforesaid protein and 0.01-99.99% of the pharmaceutical carrier, wherein the percentage is the mass percentage of the pharmaceutical composition.

Preferably, the pharmaceutical composition is administered in an effective amount, wherein the effective amount is an amount capable of alleviating or delaying the progress of a disease, degenerative or traumatic disorder. The effective amount can be determined on an individual basis and will be based in part on the consideration of the symptoms to be treated and the outcome sought.

Based on the common knowledge in the art, aforesaid preferred conditions can be combined arbitrarily to obtain the preferable embodiments of the present invention.

The reagents and raw materials used in the present invention are commercially available.

The advantage of the present invention is that the polypeptide or derivatives thereof in the present invention can be specifically bound to TRB3, and block the interaction between TRB3 and MDM2 protein, thereby it is useful in the preparation of a medicament for treating and preventing pulmonary fibrosis. The resulting medicament has advantages of remarkable curative effect, less toxic side effects and safe use in treating pulmonary fibrosis diseases.

### Brief description of the drawings

Figure 1 is an expression profile showing the increased expression of TRB3, MDM2 and SLUG in lung tissue of mice with pulmonary fibrosis.
Figure 2 is a graph showing the interaction between TRB3 and MDM2 in the lung tissue of mice with pulmonary fibrosis. Among them, the output shows the protein content of TRB3 protein and MDM2 protein contained in the lung tissue lysate after precipitation by MDM2 antibody or control antibody IgG; the input shows the protein content of TRB3 protein and MDM2 protein contained in the initial lung tissue lysate.
Figure 3 is an expression profile showing the increased expression of SLUG protein which was induced by overexpression of TRB3 in human lung epithelial cells.

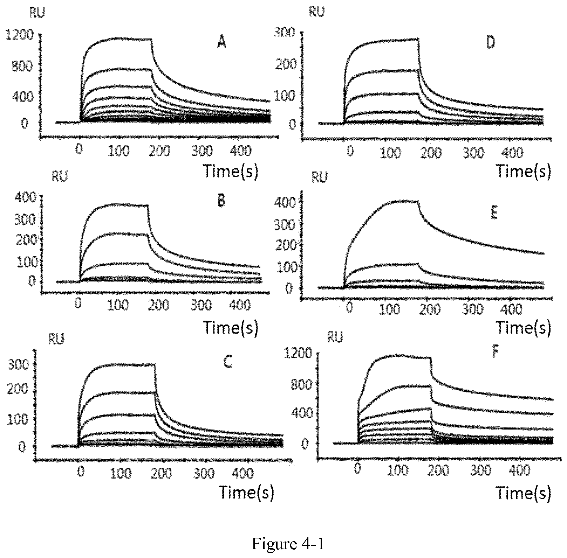

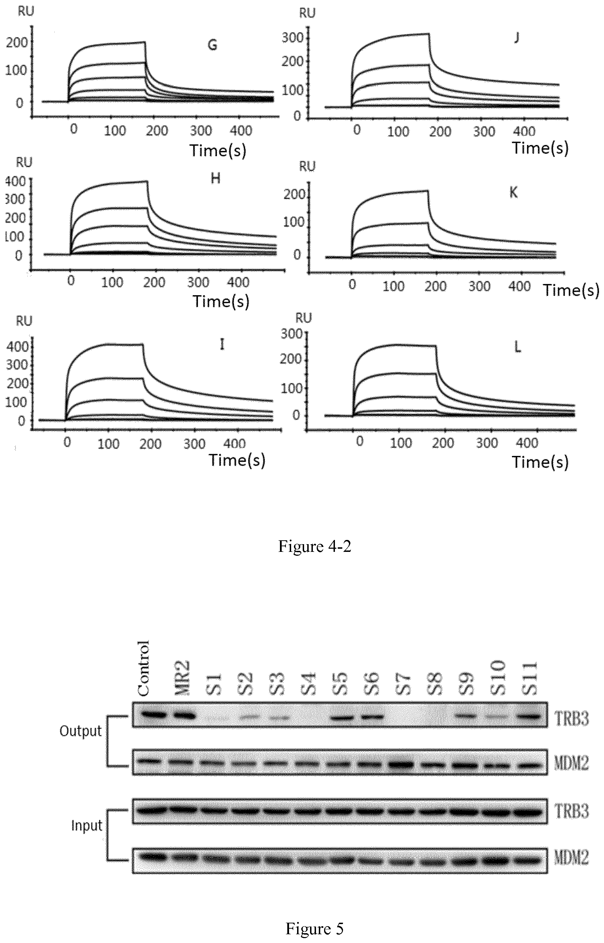

Figure 4-1 and Figure 4-2 show that the binding ability of polypeptides to TRB3 protein is confirmed by surface plasmon resonance method, wherein the polypeptides are MR2, S1, S2, S3, S4, S5, S6, S7, S8, S9, S10 and S11. "A" is the kinetic curve of polypeptide MR2 and TRB3 proteins; "B" is the kinetics curve of polypeptide S1 binding to TRB3; "C" is the kinetics curve of polypeptide S2 binding to TRB3; "D" is the kinetics curve of polypeptide S3 binding to TRB3; "E" is the kinetics curve of peptide S4 binding to TRB3 protein; "F" is the kinetic curve of polypeptide S5 binding to TRB3 protein; "G" is the kinetic curve of polypeptide S6 binding to TRB3 protein; "H" is the kinetic curve of polypeptide S7 binding to TRB3 protein; "I" is the kinetic curve of polypeptide S8 binding to TRB3 protein; J is the kinetics curve of polypeptide S9 binding to TRB3 protein; K is the kinetic curve of polypeptide S10 binding to TRB3 protein; L is the kinetic curve of polypeptide S11 binding to TRB3 protein. Among them, the abscissa is the reaction time in seconds. The ordinate is the reaction intensity between the reaction chip surface and the polypeptide with RU as unit.
Figure 5 shows that the interaction of TRB3 and MDM2 protein is interrupted by polypeptides MR2, S1, S2, S3, S4, S5, S6, S7, S8, S9, S10 and S11. Among them, the output shows the quantity of TRB3 protein and MDM2 protein contained in the cell lysate after being precipitated by MDM2 antibody or control antibody IgG; the input shows the quantity of TRB3 protein and MDM2 protein contained in the initial cell lysate.
Figure 6-1 and Figure 6-2 show the survival rate of mice with bleomycin-induced pulmonary fibrosis, wherein the pulmonary fibrosis is reduced by polypeptide MR2, S1, S2, S3, S4, S5, S6, S7, S8, S9, S10 and S11.
Figure 7 shows that the lung weight index of mice with bleomycin-induced pulmonary fibrosis, wherein the pulmonary fibrosis is reduced by polypeptide MR2, S1, S2, S3, S4, S5, S6, S7, S8, S9, S10 and S11.

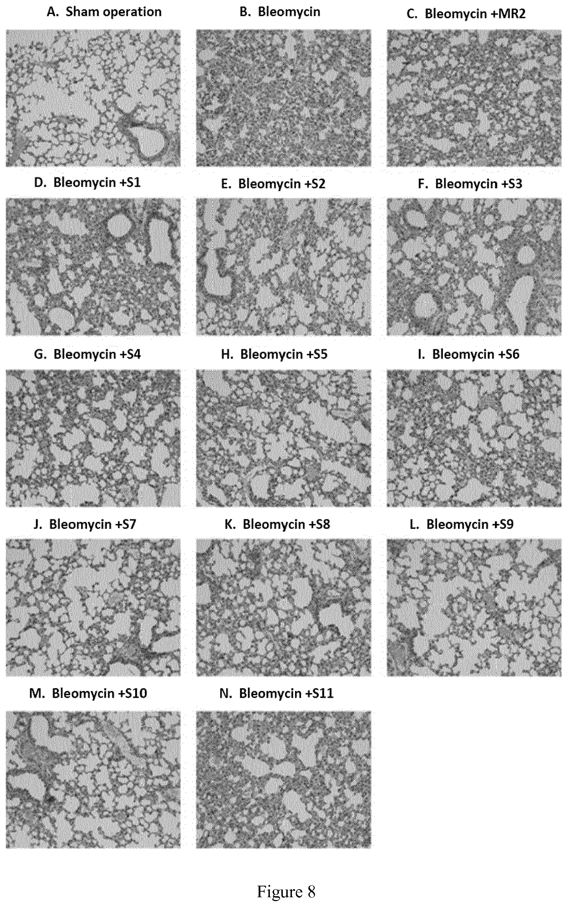

Figure 8 shows that pathological examination (HE) of mice with bleomycin-induced pulmonary fibrosis, wherein the pulmonary fibrosis is reduced by polypeptides MR2, S1, S2, S3, S4, S5, S6, S7, S8, S9, S10 and S11. Among them, "A" is sham operation group, "B" is bleomycin model group, "C" is bleomycin plus MR2 group, "D" is bleomycin plus S1 group, "E" is bleomycin plus S2 group, "F" is bleomycin plus S3 group, "G" is bleomycin plus S4 group, "H" is bleomycin plus S5 group, "I" is bleomycin plus S6 group, "J" is bleomycin plus S7 group, "K" is bleomycin plus S8 group, "L" is bleomycin plus S9 group, "M" is bleomycin plus S10 group, "N" is bleomycin plus S11 group.
Figure 9 shows that the pathological examination score of mice with bleomycin-induced pulmonary fibrosis, wherein the pulmonary fibrosis is reduced by polypeptides MR2, S1, S2, S3, S4, S5, S6, S7, S8, S9, S10 and S11.
Figure 10 shows that the hydroxyproline content in pulmonary tissue of mice with bleomycin-induced pulmonary fibrosis, wherein the pulmonary fibrosis is reduced by polypeptides MR2, S1, S2, S3, S4, S5, S6, S7, S8, S9, S10 and S11.
Figure 11 shows that the lung function of pulmonary fibrosis mice is improved by polypeptides MR2, S1, S2, S3, S4, S5, S6, S7, S8, S9, S10 and S11. Among them A is the deep inspiratory volume, B is the dynamic resistance, C is the dynamic elasticity, and D is the dynamic compliance.
Figure 12 shows the survival rate of mice with bleomycin-induced pulmonary fibrosis, wherein the pulmonary fibrosis is reduced by polypeptides S19, S20 and S21.
Figure 13 shows the lung weight index of mice with bleomycin-induced pulmonary fibrosis, wherein the pulmonary fibrosis is reduced by polypeptides S19, S20, and S21.
Figure 14 is a histopathology examination (HE) result of mice with bleomycin-induced pulmonary fibrosis, wherein the pulmonary fibrosis is reduced by polypeptide S19, S20 and S21. Among them, "A" is sham operation group, "B" is bleomycin model group, "C" is bleomycin plus S19 group, "D" is bleomycin plus S20 group, and "E" is bleomycin plus S21 group.
Figure 15 shows the histopathology examination score of mice with bleomycin-induced pulmonary fibrosis, wherein the pulmonary fibrosis is reduced by polypeptides S19, S20, and S21.
Figure 16 shows that polypeptides S19, S20, and S21 reduce hydroxyproline content in pulmonary tissue of mice with bleomycin-induced pulmonary fibrosis.
Figure 17 shows that the lung function of mice with pulmonary fibrosis is improved by polypeptides S19, S20 and S21. Among them "A" is the volume of deep inspiratory, "B" is the dynamic resistance, "C" is the dynamic elasticity, and "D" is the dynamic compliance.

### Detailed description of the preferred embodiment

The present disclosure is not to be limited in scope by the following embodiments described which are intended as single illustrations of individual aspects of the disclosure, and any functionally equivalent compositions or methods are within the scope of this disclosure. Experimental methods without specifying specific conditions in the following embodiments are selected according to conventional methods and conditions, or according to the product specifications.

The PBS solution described in the examples refers to a phosphate buffer solution with a concentration of 0.1 M and a pH of 7.2.

The room temperature described in the examples is a room temperature conventional in the art, preferably 15-30°C.

The experimental results were expressed as mean ± standard error. Through parameter or non-parametric variance test, after comparison, it is considered to have significant difference when p<0.05, and extremely significant difference when p<0.01.

### Example 1 Synthesis of a polypeptide

The amino acid sequence of polypeptide MR2 is shown in SEQ ID No. 12 of the Sequence Listing. Polypeptide MR2 was synthesized and purified by Beijing SBS Genetech Co., Ltd.

Two unnatural amino acids S-Pentenyl alanines (S5) were introduced for solid-phase polypeptide chain synthesis. After the solid-phase polypeptide chain was synthesized, olefin ring closing metathesis (RCM) reaction was conducted using ruthenium as a catalyst and then the target polypeptide is obtained. Finally, the target polypeptide was cleaved from the resin for further purification. The steps of synthesis and purification of the aforesaid solid phase polypeptide chain were completed by Chinese Peptide Company. Among them, two S-Pentenyl alanines were inserted at i^{th} and i+4^{th} positions of the amino acid sequence of polypeptide MR2, thereby obtaining modified polypeptides of different sequences (amino acid sequences of which are shown as SEQ ID No. 1-11 in the Sequence Listing). The specific insertion sites are as follows:
S1: S5-Leu-Met-Ala-S5-Phe-Thr-Cys-Ala-Lys-Lys-Leu-Lys-Lys-Arg
S2: His-S5-Met-Ala-Cys-S5-Thr-Cys-Ala-Lys-Lys-Leu-Lys-Lys-Arg
S3: His-Leu-S5-Ala-Cys-Phe-S5-Cys-Ala-Lys-Lys-Leu-Lys-Lys-Arg
S4: His-Leu-Met-S5-Cys-Phe-Thr-S5-Ala-Lys-Lys-Leu-Lys-Lys-Arg
S5: His-Leu-Met-Ala-S5-Phe-Thr-Cys-S5-Lys-Lys-Leu-Lys-Lys-Arg
S6: His-Leu-Met-Ala-Cys-S5-Thr-Cys-Ala-S5-Lys-Leu-Lys-Lys-Arg
S7: His-Leu-Met-Ala-Cys-Phe-S5-Cys-Ala-Lys-S5-Leu-Lys-Lys-Arg
S8: His-Leu-Met-Ala-Cys-Phe-Thr-S5-Ala-Lys-Lys-S5-Lys-Lys-Arg
S9: His-Leu-Met-Ala-Cys-Phe-Thr-Cys-S5-Lys-Lys-Leu-S5-Lys-Arg
S10: His-Leu-Met-Ala-Cys-Phe-Thr-Cys-Ala-S5-Lys-Leu-Lys-S5-Arg
S11: His-Leu-Met-Ala-Cys-Phe-Thr-Cys-Ala-Lys-S5-Leu-Lys-Lys-S5

### Example 2 Expression of TRB3, MDM2 and SLUG proteins in lung tissue of mice with pulmonary fibrosis were detected by Western-Blot

### 1. Preparation of animal models

Bleomycin with batch number 640412 was purchased from Nippon Kayaku.

The compounds used in Example 2 were purchased from Sigma unless otherwise specified.

SPF C57BL/6 mice (male, 6-8 weeks old, 16-18 g) were purchased from Beijing Vital River Laboratory Animal Technology Co., Ltd.

Male C57BL/6 mice were fasted overnight, then anesthetized with 45 mg/kg sodium pentobarbital by injection intraperitoneally (i.p.), and intratracheally injected with bleomycin at a dose of 3 U/kg. The specific operation steps are as follows: cut the neck skin of the mouse with as little trauma as possible, expose the trachea with the assistance of elbow ophthalmic forceps, puncture the trachea and inject about 50 µL of bleomycin into the trachea with a microsyringe, rotate the mouse quickly and stand it upright for 5 minutes in order to make bleomycin evenly enter the left and right lobe, thereby constructing an animal model. The whole operation was performed at a surgical table at about 60 °C. Mice in the sham operation group were intratracheally injected with an equal amount of physiological saline for injection.

The mice of the animal model constructed above were feed in SPF-level animal room, and sacrificed after fibrosis pathological changes occurred in the lungs on the 10th day, and the lung tissues of the mice were taken for subsequent experiments. The mice in the sham operation group were also feed in the SPF-level animal room, and sacrificed on the 10th day. Lung tissues were taken for subsequent experiments.

### 2. Expression of TRB3, MDM2 and SLUG protein in lung tissue of mice with pulmonary fibrosis were detected by Western-Blot

Appropriate amount of lung tissue was taken from the animal model obtained in step 1, then the lung tissue was added with lysis buffer [containing 0.1 mM ethylenediaminetetraacetic acid (EDTA), 0.1 mM Ethylenebis (oxyethylenenitrilo) tetraacetic acid (EGTA), 10 mM KCl, 10 mM 4-(2-hydroxyerhyl)piperazine-1-erhanesulfonic acid (HEPEs), 50 mM NaF, 0.1 M Na₃VO₄, 0.1 M Na₃PO₄, 1 mM/L aprotinin, 1 mM/L trypsin inhibitor, 1 mM/L Phenylmethanesulfonyl fluoride (PMSF), 1 mM/L leupeptins and 1 mM/L dithiothreitol (DTT)] and homogenized. Subsequently the resulting lung tissue was placed on ice for 15 minutes with occasional oscillation, then ethyl phenyl polyethylene glycol (NP-40) was rapidly added in and mixed well, and the mixture was centrifuged at 4 °C, 12000 rpm for 5 min. Supernatant was taken, and protein concentration was determined by Coomassie Brilliant Blue method. The concentration of protein was adjusted to the same for Western Blot analysis.

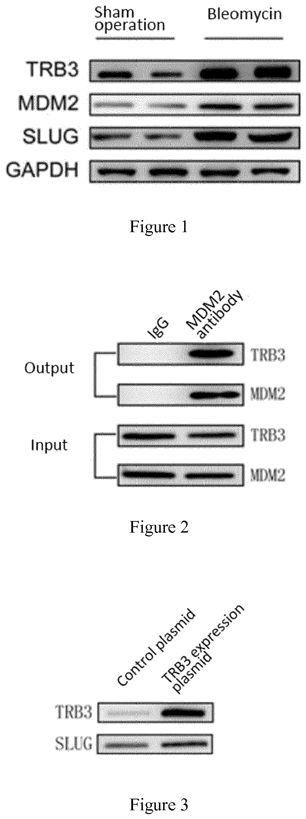

TRB3, MDM2 and SLUG was detected and analyzed using Western Blot. The color was developed by Amersham color development solution (NBT/BCIP staining kit IK5030, purchased from Huamei Co., Ltd.), and the optical density value of each band was measured by Western-blot analysis software (gelPro32). The results shown in Fig. 1 indicates that the TRB3, MDM2, and SLUG proteins in the lung tissue of mice with pulmonary fibrosis are highly expressed.

### Example 3 Binding of MDM2 to TRB3 protein in tissue of pulmonary fibrosis was verified by co-Immunoprecipitation

The reagents used for co-immunoprecipitation are as follows:
Lysate A: 0.6057g Tris base, 1.7532g NaCl, 0.1017g MgCl₂•6H₂O, 0.0742g EDTA, 10mL glycerol and 10mL 10% (v/v) NP40 were weighed, and deionized water were added to 150mL. The pH was adjusted to 7.6 with HCl, and the constant volume was adjusted to 191 mL to obtain mixture. The mixture was thoroughly mixed, filtered through a 0.45 µm filter, and stored at 4 °C.
Lysate B: 200 µL of 2M β-glycerophosphate, 4 mL of 2.5 M NaF, 2 mL of 100 mM NaVO₃, 2 mL of 100 mM PMSF, 200 µL of 1 M DTT, 200 µL of 1 mg/mL Leu, 200 µL of 1 mg/mL Pep, and 200 µL of 1 mg/mL Apr were weighed to obtain mixture with a total volume of 9 mL, and stored at -20 °C. When used, the lysate B solution was thawed and added to the lysate A solution at a volume ratio of 1:100 (the lysate B solution: the lysate A solution) to obtain a mixture, and the mixture was mixed thoroughly, thus obtaining the cell lysate.

Co-immunoprecipitate lotion comprises 1% (v/v) NP40, 150 mM NaCl, 20 mM HEPES, 10% (v/v) pH 7.5 glycerol and 1 mM EDTA.

Protein A/G Plus-Agarose was purchased from Santa Cruz, USA.

The specific steps are as follows:
(1) Appropriate amount of lung lobar tissue was taken from the animal model obtained in Step 2 of the above Example 2, and weighed.
(2) The lung tissue weighed in step (1) was lysed using the co-immunoprecipitate lysate, and about 10 mg of cell total protein was harvested. The lung tissue protein of the animal model and the sham operation group were both adjusted to a concentration of 10 µg/mL. 200 µg of lung tissue protein of the animal model and the sham operation group were taken as input groups, and used as a control.
(3) 2 µg of MDM2 antibody (purchased from Abeam, catalog number ab16895) and common IgG antibody (purchased from Cell Signaling, catalog number 2729), which has same species with the MDM2 antibody , were added to same amount of 9800 µg remaining protein in each group, and simultaneously 10 µL of Protein A/G Plus-Agarose (purchased from Santa Cruz, catalog number SC-2003) was added, and resuspended thoroughly and shaken slowly at 4°C overnight. Mixture was centrifuged at 4 °C, 3000 rpm for 5 min, and the supernatant was carefully aspirated. 0.5 mL of the co-immunoprecipitate lysate was added and mixed well. Stood resulting mixture still on ice for 1min, then the mixture was centrifuged at 4 °C, 3000 rpm for 30 seconds, and the supernatant was carefully aspirated. Then the washing was repeated 5 times and the resulting mixture was allowed to stand for 5 min before the last centrifugation. The supernatant was carefully aspirated, 30 µL of 2×SDS gel loading buffer was added and mixed well. Resulting mixture was denatured at 95°C for 3 min, and rapidly transferred to an ice bath for cooling. Then centrifugation at 12000 rpm for 2 min at room temperature was performed, and the supernatant was the precipitated protein sample. A portion of the protein sample was taken for SDS-polyacrylamide gel electrophoresis.

Results shown in Fig. 2 indicates that TRB3 protein and MDM2 protein are bound to each other in the tissue of pulmonary fibrosis. Among them, "input" used the cell lysate described above; "input" represents the initial content of TRB3 and MDM2 in the protein sample, that is, the content of TRB3 protein and MDM2 protein in the protein sample before precipitation by MDM2 antibody or control antibody IgG (since the MDM2 antibody is an IgG-type antibody, the IgG antibody was selected as a control). The results shows that the content of TRB3 protein and MDM2 protein in the lysate of the input group were the same.

"Output" represents the TRB3 and MDM2 content in the protein sample after precipitation by MDM2 antibody or control antibody IgG. As the IgG antibody was used as a control antibody for the MDM2 antibody, and the MDM2 protein could not be precipitated by IgG antibody, so the western blot lane of MDM2 shows blank in the IgG antibody-treated protein samples. As an antibody of experimental group, MDM2 antibody can bind to the MDM2 protein and precipitates it. Therefore, the result of the protein sample treated with MDM2 antibody shows that the western blot lane of MDM2 is black. It is precisely because of the interaction between the MDM2 protein and the TRB3 protein that the MDM2 protein can also precipitate the TRB3 protein when the MDM2 protein is precipitated, so the western blot lane of TRB3 protein in the cell lysate treated with the MDM2 antibody is shown as black. Since the IgG antibody could not precipitate the MDM2 protein, and the TRB3 protein, which is interacted with MDM2, could not be precipitated as well, so the western blot lane of TRB3 in the IgG antibody-treated cell lysate is blank. These results fully demonstrate that the TRB3 protein can interact with the MDM2 protein directly.

### Example 4 Increase of SLUG in lung epithelial cells was induced by TRB3

(1) 400 µL of nuclease-free water was added to the tube, shaken for 10 seconds, and the transfection reagent (lipofectamine 2000, purchased from Invitrogen) was diluted.
(2) Transfection reagent and plasmid DNA [control plasmid (purchased from Ao Ruidong, model PS100001); TRB3 overexpression plasmid (purchased from Ao Ruidong, model RC206687)] were mixed at a ratio [amount of the transfection reagent (mL) to the mass of the DNA (mg)] of 1:2, and allowed to stand at room temperature for 15 minutes to obtain a mixed solution.
(3) M199 medium in the culture plate of BEAS-2B cells (purchased from the Institute of Basic Medical Sciences, Chinese Academy of Medical Sciences) was aspirated and washed once with PBS.
(4) 400 µL of mixed solution was added, and the cells were cultured at 37 °C in an incubator for 24 hours.
(5) The transfected BEAS-2B cells were lysed using RIPA lysate (purchased from Beyotime, model P0013B), and the expression of intracellular SLUG protein was measured by Western-blot method in Example 3.

Results are shown in Fig. 3. Fig. 3 illustrates that the expression of Slug in BEAS-2B cells overexpressing TRB3 is significantly increased.

### Example 5 Binding ability of peptide S1-S11 to TRB3 protein were detected by surface plasmon resonance

The surface plasmon resonance experiment was carried out in a surface plasmon resonator Biacore T200, and the procedure was carried out in accordance with the instructions of the plasma resonator Biacore T200. Specific steps are as follows:
1. Purified TRB3 protein (purchased from RD) was coupled to a CM5 chip (purchased from GE) by amidogen, and the unbound protein was eluted at a flow rate of 10 µL/min. The surface of the chip was equilibrated for 2 hours. Among them, the specific steps of amidogen coupling, elution and equilibration refer to the relevant instruction of GE's CM5 chip.
2. 250 µL of the S1-S11 and MR2 polypeptide fragments prepared in Example 1 with different concentrations (800, 400, 200, 50, 12.5, 6.25, and 3.125 nM) were automatically injected, and the entire surface plasmon resonance experiment was carried out at 25 °C. The buffer used was HBS-EP buffer [0.01 M HEPES, 0.15 M NaCl, 3 mM EDTA and 0.005% (w/w) surfactant TWEEN20]. Biacore T200's built-in analysis software was used to simulate the binding curves of different concentrations of peptides to TRB3. The results are shown in Fig. 4-1, 4-2 and Table 1. Fig. 4-1, 4-2 and Table 1 illustrate that peptides MR2, S1, S2, S3, S4, S5, S6, S7, S8, S9, S10 and S11 can all interacted with the TRB3 protein.

**Table 1 Affinity test of peptides S1-S11 and MR2 with TRB3 protein**

| Name of polypeptide | Affinity constant (KD) with TRB3 protein |
|---|---|
| MR2 | 4.33×10⁻⁸ M |
| S1 | 2.02×10⁻⁸ M |
| S2 | 1.91×10⁻⁸ M |
| S3 | 4.27×10⁻⁸ M |
| S4 | 9.63×10⁻⁹ M |
| S5 | 1.15×10⁻⁸ M |
| S6 | 4.96×10⁻⁸ M |
| S7 | 8.95×10⁻⁹ M |
| S8 | 3.59×10⁻⁸ M |
| S9 | 3.71×10⁻⁸ M |
| S10 | 2.93×10⁻⁸ M |
| S11 | 3.77×10⁻⁸ M |

### Example 6 α-helix ratio of polypeptides S1-S11 were detected by circular dichroism

The α-helix ratio of the polypeptide was measured by a circular dichromatograph (purchased from Jasco, Japan). The polypeptides MR2, S1, S2, S3, S4, S5, S6, S7, S8, S9, S10 and S11 prepared in Example 1 were dissolved in PBS solution, and the working concentration of the circular dichromatograph was adjusted to 1 mg/mL. The results are shown in Table 2. Table 2 shows that the α-helix ratio of the polypeptides S1, S2, S3, S4, S5, S6, S7, S8, S9, S10 and S11 were significantly higher than that of polypeptide MR2. Among them, the α-helix ratio refers to the percentage of the number of peptide segments of the polypeptide that maintains the alpha helix of the secondary structure in the peptide segments of the total polypeptide.

**Table 2α-helix ratio of polypeptides were determined by circular dichroism**

| Name of polypeptide | MR2 | S1 | S2 | S3 | S4 | S5 | S6 | S7 | S8 | S9 | S10 | S11 |
|---|---|---|---|---|---|---|---|---|---|---|---|---|
| α-helicity (%) | 0.0 | 39.5 | 25.7 | 42.1 | 33.6 | 69.1 | 36.4 | 51.0 | 29.1 | 44.0 | 31.5 | 37.8 |

### Example 7 The inhibition of S1-S11 and MR2 polypeptides on the binding of protein MDM2 to TRB3 at the cellular level were verified by method of co-immunoprecipitation

1. Human lung epithelial BEAS-2B cells (purchased from Institute of Basic Medical Sciences, Chinese Academy of Medical Sciences) were seeded in a 90 mm² culture dish. After the cells were adhered to the wall of culture dish, the TRB3 expression plasmid were transferred to the cells according to the method described in Example 5. After 24 hours, 1 mg/mL of the polypeptides MR2, S1, S2, S3, S4, S5, S6, S7, S8, S9, S10 or S11 prepared in Example 1 were added respectively, and the cells were incubated for 12 hours in a 37 °C incubator and then harvested.
2. Co-immunoprecipitation with MDM2 antibody was performed according to the method described in Example 3, followed by detection using TRB3 antibody.

Results are shown in Fig. 5. The results in Fig. 5 demonstrate that the interference of peptides S1-S11 on TRB3/MDM2 protein interaction is significantly stronger than that of MR2.

### Example 8 The effects of peptides S1-S11 on mortality in mice with pulmonary fibrosis were determined

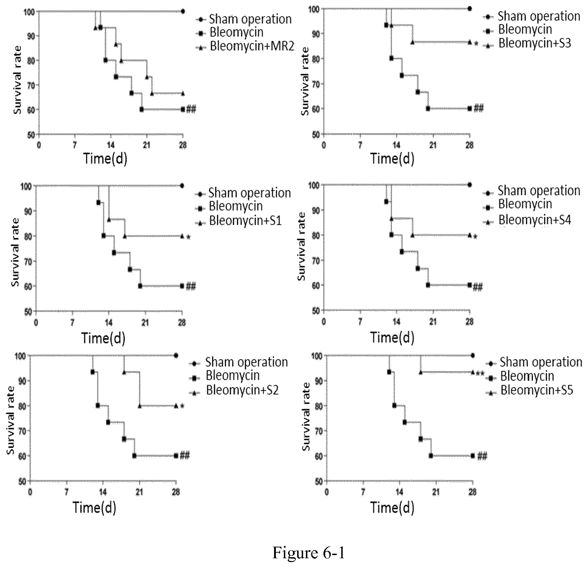

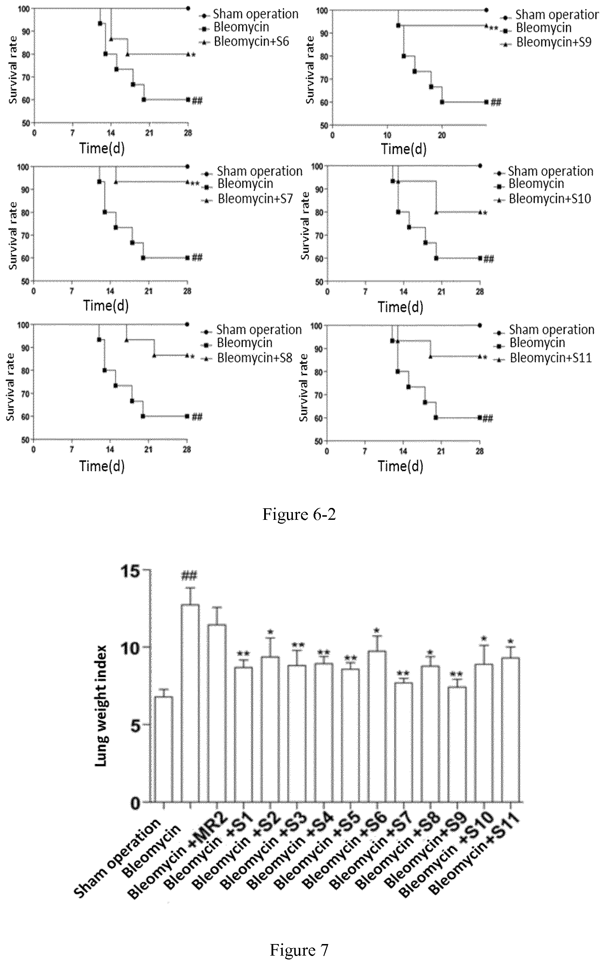

The animal model with pulmonary fibrosis was prepared according to the method described in Example 2. The peptide therapy was started from the 10th day after modeling (the treatment groups are shown in Table 3), and the time was calculated from the 10th day after modeling to count the mortality rate. The death situation of each group of experimental animals was counted and calculated daily. If a group of animals do not die, the survival rate is 100%, and if all animals in a group died, the survival rate is 0%. The survival rate is the percentage of the number of mice that survived accounting for the number of mice in the whole group. Results are shown in Fig. 6-1, Fig. 6-2 and Table 4. Fig. 6-1 and Fig. 6-2 show that compared with the sham operation group, the survival rate of the model group is significantly reduced. After drug treatment, the S1-S11 administration group can significantly improve the survival rate of mice with pulmonary fibrosis. In Fig. 6-1 and Fig. 6-2, "##" represents that P value is less than 0.01 when the survival rate of administration group was compared with that of the sham operation group; "*" represents that P value is less than 0.05 when the survival rate of administration group was compared with that of the bleomycin group; "**" represents that P value is less than 0.01 when the survival rate of administration group was compared with that of bleomycin group; and "i.p." refers to intraperitoneal injection. The above results indicate that the polypeptides of the present invention can effectively reduce the mortality rate of mouse in pulmonary fibrosis model, and possess the advantages of less toxic side effects and safer use.

**Table 3 Grouped administration of animal model with pulmonary fibrosis after modeling**

| Groups | Number | Dosage and administration schedule | Administration route | Solvent |
|---|---|---|---|---|
| sham operation group | 15 | 2 mg/kg Deionized water; From the 10th to the 28th day after modeling, twice a week. | i.p. | PBS |
| bleomycin group | 20 | 2 mg/kg bleomycin; From the 10th to the 28th day after modeling, twice a week. | i.p. | PBS |
| MR2 administration group | 20 | 2 mg/kg MR2; From the 10th to the 28th day after modeling, twice a week. | i.p. | PBS |
| S1 administration group | 20 | 2 mg/kg S1; From the 10th to the 28th day after modeling, twice a week. | i.p. | PBS |
| S2 administration group | 20 | 2 mg/kg S2; From the 10th to the 28th day after modeling, twice a week. | i.p. | PBS |
| S3 administration group | 20 | 2 mg/kg S3; From the 10th to the 28th day after modeling, twice a week. | i.p. | PBS |
| S4 administration group | 20 | 2 mg/kg S4; From the 10th to the 28th day after modeling, twice a week. | i.p. | PBS |
| S5 administration group | 20 | 2 mg/kg S5; From the 10th to the 28th day after modeling, twice a week. | i.p. | PBS |
| S6 administration group | 20 | 2 mg/kg S6; From the 10th to the 28th day after modeling, twice a week. | i.p. | PBS |
| S7 administration group | 20 | 2 mg/kg S7; From the 10th to the 28th day after modeling, twice a week. | i.p. | PBS |
| S8 administration group | 20 | 2 mg/kg S8; From the 10th to the 28th day after modeling, twice a week. | i.p. | PBS |
| S9 administration group | 20 | 2 mg/kg S9; From the 10th to the 28th day after modeling, twice a week. | i.p. | PBS |
| S10 administration group | 20 | 2 mg/kg S10; From the 10th to the 28th day after modeling, twice a week. | i.p. | PBS |
| S11 administration group | 20 | 2 mg/kg S11; From the 10th to the 28th day after modeling, twice a week. | i.p. | PBS |

**Table 4. Effect of polypeptide S1-S11 on mortality rate in mice with pulmonary fibrosis**

| Groups | Survival rate (%) | | | |
|---|---|---|---|---|
| | 7 days | 14 days | 21 days | 28 days |
| sham operation group | 100 | 100 | 100 | 100 |
| bleomycin group | 100 | 80 | 60 | 60 |
| MR2 administration group | 100 | 93.33 | 73.33 | 66.67 |
| S1 administration group | 100 | 86.67 | 80 | 80 |
| S2 administration group | 100 | 100 | 80 | 80 |
| S3 administration group | 100 | 93.33 | 86.67 | 86.67 |
| S4 administration group | 100 | 86.67 | 80 | 80 |
| S5 administration group | 100 | 100 | 93.33 | 93.33 |
| S6 administration group | 100 | 86.67 | 80 | 80 |
| S7 administration group | 100 | 100 | 93.33 | 93.33 |
| S8 administration group | 100 | 100 | 93.33 | 86.67 |
| S9 administration group | 100 | 93.33 | 93.33 | 93.33 |
| S10 administration group | 100 | 93.33 | 80 | 80 |
| S11 administration group | 100 | 93.33 | 86.67 | 86.67 |

### Example 9 Effects of polypeptides S1-S11 on lung weight index in mice with pulmonary fibrosis were detected

Pulmonary fibrosis model was prepared according to the method described in Example 2, and administered in groups according to Table 3. After 28 days, the lungs of the mice were finely stripped and weighed, and a lung weight index was obtained from dividing the lung weight (mg) by the body weight (g) of the mice. Results are shown in Fig. 7 and Table 5. As can be seen from Fig. 7, compared with the sham operation group, the lung weight index of the mice was significantly increased after administration of bleomycin. In Fig. 7, "##" represents that P value is less than 0.01 when the lung weight index of administration group was compared with that of the sham operation group; "*" represents that P value is less than 0.05 when the lung weight index of administration group was compared with that of the bleomycin group; "**" represents that P value is less than 0.01 when the lung weight index of administration group was compared with that of bleomycin group.

**Table 5 Effect of polypeptides S1-S11 on lung weight index in mice with pulmonary fibrosis**

| Groups | Lung weight index |
|---|---|
| sham operation group | 6.7875 |
| bleomycin group | 12.7375 |
| MR2 administration group | 11.4625 |
| S1 administration group | 8.675 |
| S2 administration group | 9.35 |
| S3 administration group | 8.8 |
| S4 administration group | 8.925 |
| S5 administration group | 8.575 |
| S6 administration group | 9.725 |
| S7 administration group | 7.675 |
| S8 administration group | 8.775 |
| S9 administration group | 7.4125 |
| S10 administration group | 8.8875 |
| S11 administration group | 9.2875 |

### Example 10 Effects of polypeptide S1-S11 on pathomorphology of bleomycin-induced pulmonary fibrosis was determined

General tissue changes and tissue products can be visualized by HE staining method, namely Hematoxylin and Eosin staining method, which is the most commonly used staining method for morphology. Among them, hematoxylin dyeing solution is an alkaline dyeing solution, which mainly makes the chromatin in the nucleus and the ribosome in the cytoplasm purple blue; eosin is an acid dye, which mainly makes the components in the cytoplasm and extracellular matrix red. Pulmonary fibrosis models were made according to the method described in Example 2 and administered in groups according to Table 3. After 28 days, the right lower lobe of lung tissue of the experimental animals was taken, and fixed with 4% (v/v) paraformaldehyde and embedded in paraffin. Section was performed on the largest cross-section of the paraffin block in which the lung tissue embedded, and basic pathological changes was observed through HE staining. The results are shown in Fig. 8. Fig. 8 shows that the HE stained lung tissue of sham-operated mice is clearly visible, the alveolar structure is intact, and no pathological changes of inflammation and fibrosis are observed. The bleomycin group showed obvious inflammation in the lungs of mice, the inflammatory cells infiltrated in a large amount, and the lung tissue structure was severely damaged. The administration of polypeptides S1-S11 can reduce lung inflammation caused by bleomycin, and effectively ameliorate lung injury and restore normal lung structure.

The inflammatory grading was observed according to the results of HE staining, and the criteria were as follows (grade 0-5): grade "0": normal tissue; grade "1": extremely small inflammatory changes; grade "2": mild to moderate inflammatory changes without apparent destruction of lung tissue structure; grade "3": moderate inflammatory injury (thickening of alveolar diaphragm); grade "4": moderate to severe inflammatory injury with formation of tissue mass, or the normal structure of lung tissue was destroyed by localized pneumonia area; grade "5": severe inflammatory injury, severe destruction of the localized lung tissue structure, resulting in closure of lumen.

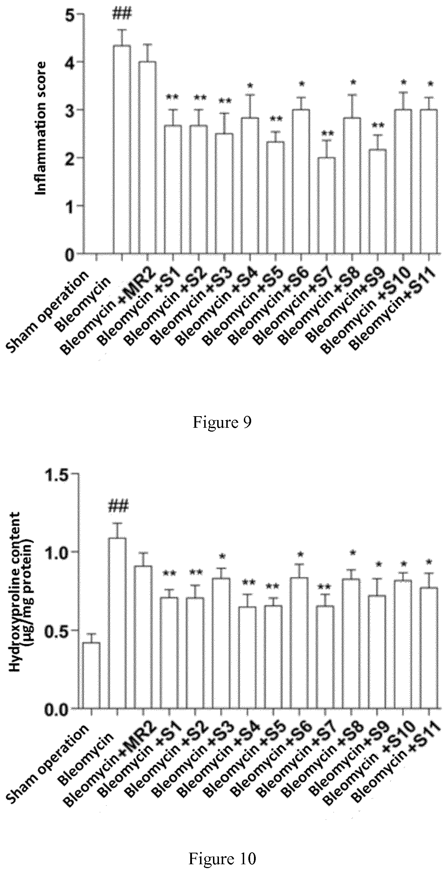

The results are shown in Figs. 9 and Table 6. The results showed that after administration of bleomycin, significant inflammation occurred in the lungs of mice compared to the sham-operated group. Administration of polypeptides S1-S11 even can significantly reduce lung inflammation caused by bleomycin. In Fig. 9, "##" represents that P value is less than 0.01 when inflammation score of the administration group was compared with that of the sham operation group; "*" represents that P value is less than 0.05 when inflammation score of the administration group was compared with that of the bleomycin group; "**" represents that P value is less than 0.01 when inflammation score of the administration group was compared with that of bleomycin group.

**Table 6 Inflammatory score of peptides S1-S11 on bleomycin-induced pulmonary fibrosis**

| Groups | Inflammatory score |
|---|---|
| sham operation group | 0 |
| bleomycin group | 4.333333 |
| MR2 administration group | 4 |
| S1 administration group | 2.666667 |
| S2 administration group | 2.666667 |
| S3 administration group | 2.5 |
| S4 administration group | 2.833333 |
| S5 administration group | 2.333333 |
| S6 administration group | 3 |
| S7 administration group | 2 |
| S8 administration group | 2.833333 |
| S9 administration group | 2.166667 |
| S10 administration group | 3 |
| S11 administration group | 3 |

### Example 11 Effects of polypeptides S1-S11 on hydroxyproline content in mice with pulmonary fibrosis were determined

Hydroxyproline accounts for 13.4% (w/w) of collagen, and extremely small amount of elastin, while it is absent in other proteins. Hence the collagen content was detected by hydroxyproline. Pulmonary fibrosis models were prepared according to the method described in Example 2 and administered in groups according to Table 3. The content of hydroxyproline in the lobes of left lung of the animals was measured 28 days later, and the condition of pulmonary fibrosis was evaluated.

The specific method is as follows: the mice in the animal model prepared in Example 2 was feed in a SPF animal room and treated with polypeptide according to the dosing schedule of Table 3, and all left lobes of lung of the experimental animal was taken on the 21st day after the animal model was constructed and the wet weight was recorded. Then 10% (w/w) tissue homogenate was prepared by ultrasonic homogenization using physiological saline. About 150 µL of homogenate supernatant was taken, and 500 µL of alkali hydrolysate was added (provided by Hydroxyproline alkaline hydrolysis kit of Nanjing Jiancheng Bioengineering Technology Co., Ltd.). After vortex mixing, the mixture was treated with alkaline hydrolysis for 40 min under conditions of 120 °C, 0.1Kpa. After adjusting the pH value, the volume was held constant, and the supernatant was taken after performing activated carbon treatment. The hydroxyproline was determined according to the chloramine-T method (the method of the present example refers to the instruction provided with kit, which was purchased from Nanjing Jiancheng Bioengineering Technology Co., Ltd.). The results are shown in Fig. 10 and Table 7. As can be seen from Fig. 10, compared with the sham operation group, the hydroxyproline content of the bleomycin group was significantly increased, indicating that the pathological changes of fibrosis were severe. Administration of S1-S11 can significantly reduce the content of hydroxyproline in the lungs of mice with pulmonary fibrosis. In Fig. 10, "##" represents that P value is less than 0.01 when comparing the content of hydroxyproline in the administration group with that in the sham operation group; and "*" represents that P value is less than 0.05 and"**" represents that P value is less than 0.01 when comparing the content of hydroxyproline in the administration group with that in bleomycin group.

**Table 7 Effects of polypeptides S1-S11 on hydroxyproline content in mice with pulmonary fibrosis**

| groups | hydroxyproline content (µg/mg protein) |
|---|---|
| sham operation group | 0.42125 |
| bleomycin group | 1.0875 |
| MR2 administration group | 0.90875 |
| S1 administration group | 0.70875 |
| S2 administration group | 0.70625 |
| S3 administration group | 0.82875 |
| S4 administration group | 0.65 |
| S5 administration group | 0.6575 |
| S6 administration group | 0.83375 |
| S7 administration group | 0.655 |
| S8 administration group | 0.825 |
| S9 administration group | 0.72125 |
| S10 administration group | 0.81625 |
| S11 administration group | 0.7725 |

### Example 12 Effects of polypeptides S1-S11 on pulmonary function in mice with pulmonary fibrosis were determined

Pulmonary function is a gold indicator for clinical detection of pulmonary fibrosis in patients. The decline of lung function is generally accompanied by an aggravation of fibrosis, while the improvement of lung function generally represents the recovery of lung tissue structure. Pulmonary fibrosis models were prepared according to the method described in Example 2 and administered in groups according to Table 3. On the 28th day after the construction of the animal model, the mice were anesthetized with sodium pentobarbital (45 mg/kg, i.p.). Lung function was tested by the Flexivent Small-animal respiratory mechanics and the testing methods were TLC and SnapShots (Testing method refers to Lv X, Wang X, Li K, et al. Rupatadine Protects against Pulmonary Fibrosis by Attenuating PAF-Mediated Senescence in Rodents[J]. PloS one, 2013, 8(7): e68631*).*

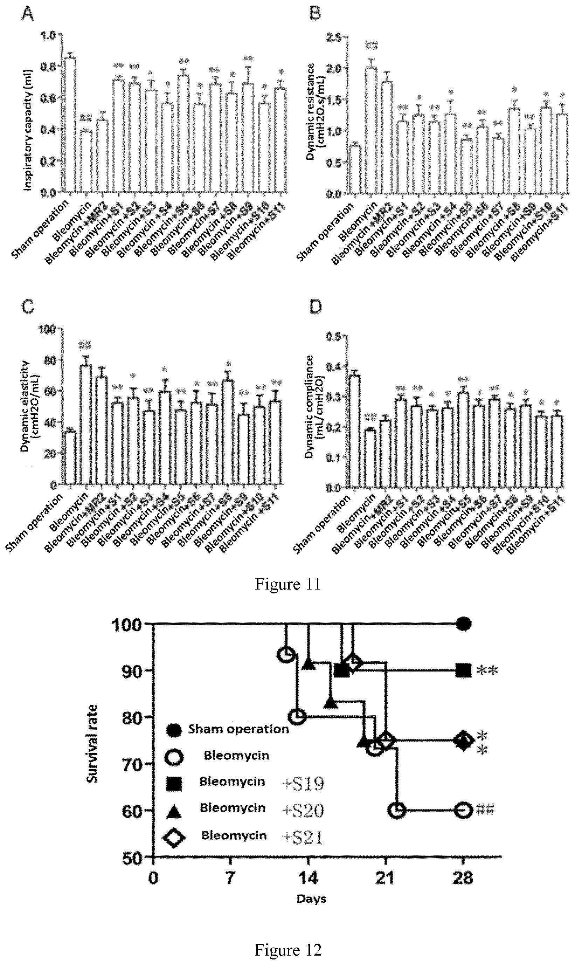

The results can be seen in Fig. 11 and Table 8, wherein "A" represents inspiratory capacity, "B" represents dynamic resistance, "C" represents dynamic elasticity and "D" represents dynamic compliance. Fig. 11 shows that, compared to the sham operation group, the inspiratory capacity of mice with bleomycin-induced pulmonary fibrosis was significantly reduced, the dynamic resistance and the dynamic elasticity of lungs were increased, and the compliance was significantly reduced. Pulmonary function was significantly restored after treatment with peptides S1-S11. In Fig. 11, "##" represents that P value is less than 0.01 when comparing the effects of administration groups with that of the sham operation group; "*" represents that P value is less than 0.05 and "**" represents that P value is less than 0.01 when comparing the effects of administration groups with that of the bleomycin group.

**Table 8 Effects of polypeptides S1-S11 on pulmonary function in mice with pulmonary fibrosis**

| Groups | Inspiratory capacity (mL) | Dynamic resistance (cmH₂O.s/Ml) | Dynamic elasticity (cmH₂O./mL) | Dynamic compliance (mL/cmH₂O) |
|---|---|---|---|---|
| sham operation group | 0.852 | 0.758333 | 33.53701 | 0.368333 |
| bleomycin group | 0.382 | 1.996667 | 76.16667 | 0.188333 |
| MR2 administration group | 0.456 | 1.775 | 68.83333 | 0.22 |
| S1 administration group | 0.71 | 1.15 | 52.16667 | 0.288333 |
| S2 administration group | 0.688 | 1.25 | 55.33333 | 0.268333 |
| S3 administration group | 0.646 | 1.143333 | 47 | 0.255 |
| S4 administration group | 0.564 | 1.266667 | 59.33333 | 0.261667 |
| S5 administration group | 0.738 | 0.85 | 47.5 | 0.311667 |
| S6 administration group | 0.558 | 1.066667 | 52.16667 | 0.268333 |
| S7 administration group | 0.684 | 0.883333 | 51 | 0.29 |
| S8 administration group | 0.626 | 1.35 | 66.5 | 0.258333 |
| S9 administration group | 0.686 | 1.033333 | 44.5 | 0.27 |
| S10 administration group | 0.562 | 1.366667 | 49.5 | 0.233333 |
| S11 administration group | 0.658 | 1.266667 | 53 | 0.235 |

The results of the above example show that the polypeptides of the present invention have a remarkable anti-pulmonary fibrosis effect and can be used as an active ingredient for the preparation of anti-pulmonary fibrosis drug.

### Example 13 Synthesis of polypeptides derivatives S19-S21 (chimeric polypeptide formed by polypeptide MR2 and cell membrane penetrating polypeptide)

S19 [chimeric polypeptide Pep2-MR2 (amino acid sequence of which is shown in SEQ ID No. 19 of the Sequence Listing)], S20 [chimeric polypeptide TAT-MR2 (amino acid sequence of which is shown in SEQ ID No. 20 of the Sequence Listing)] and S21 [chimeric polypeptide Antp-MR2 (amino acid sequence of which is shown in SEQ ID No. 21 of the Sequence Listing)] were synthesized and purified by Beijing SBS Genetech Co., Ltd.

### Example 14 Determination of the effects of S19-21 on mortality in mice with pulmonary fibrosis

The animal model of pulmonary fibrosis was prepared according to the method described in Example 2. The polypeptide treatment was started from the 10th day after modeling (the treatment groups are shown in Table 9), and the time was calculated from the 10th day after modeling to count the mortality rate. The mortality rate of each group of experimental animals was recorded and calculated daily. If none of the animals in a group died, the survival rate of the group is 100%. If all the animals in a group died, the survival rate is 0%. The results are shown in Fig. 12 and Table 10. Fig. 12 illustrates that compared with the sham group, the survival rate of the model group is significantly reduced. After drug treatment, the survival rate of mice with pulmonary fibrosis in S19-S21 administration groups can be significantly improved. In Fig. 12, "##" represents that P value is less than 0.01 when the survival rate of the the administration groups were compared with that of the sham operation group; "*" represents that P value is less than 0.05 and "**" represents that P value is less than 0.01 when the survival rate of the administration groups were compared with bleomycin group. The above results indicate that the polypeptide derivatives of the present invention can effectively reduce the mortality rate of mice model with pulmonary fibrosis, and possess the advantages of less toxic side effects and safer use.

**Table 9 Grouping administration of animal model with pulmonary fibrosis after modeling**

| Groups | Number | Administration dosage and administration schedule | Administration route | Solvent |
|---|---|---|---|---|
| sham operation group | 15 | 2 mg/kg Deionized water; From the 10th to the 28th day after modeling, twice a week. | i.p. | PBS |
| bleomycin group | 20 | 2 mg/kg bleomycin; From the 10th to the 28th day after modeling, twice a week. | i.p. | PBS |
| S19 administration group | 20 | 2 mg/kg S19; From the 10th to the 28th day after modeling, twice a week. | i.p. | PBS |
| S20 administration group | 20 | 2 mg/kg S20; From the 10th to the 28th day after modeling, twice a week. | i.p. | PBS |
| S21 administration group | 20 | 2 mg/kg S21; From the 10th to the 28th day after modeling, twice a week. | i.p. | PBS |

**Table 10 Effects of S119-21 on mortality of mice with pulmonary fibrosis**

| Groups | Survival rate (%) | | | |
|---|---|---|---|---|
| | 7 days | 14 days | 21 days | 28 days |
| sham operation group | 100 | 100 | 100 | 100 |
| bleomycin group | 100 | 80 | 73.33 | 60 |
| S19 administration group | 100 | 100 | 90 | 90 |
| S20 administration group | 100 | 91.67 | 75 | 75 |
| S21 administration group | 100 | 100 | 75 | 75 |

### Example 15 Effect of S19-21 on lung weight index in mice with pulmonary fibrosis

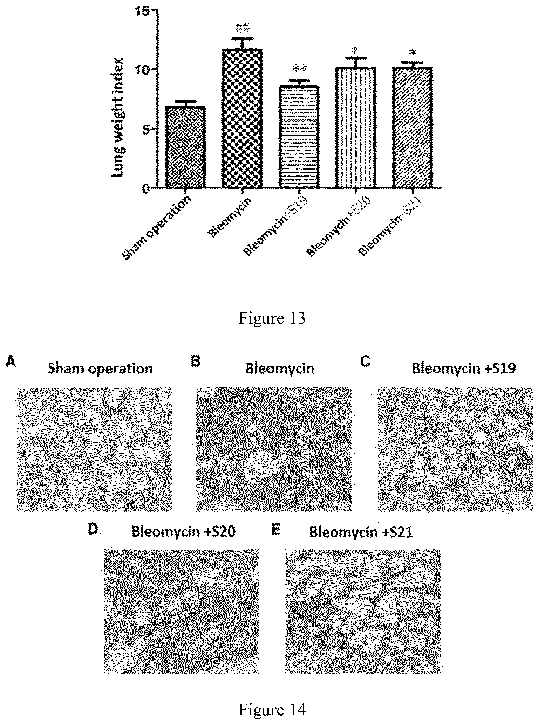

Pulmonary fibrosis models were made according to the method described in Example 2 and administered in groups according to Table 9. After 28 days, the lungs of the mice were finely stripped and weighed, and the lung weight (mg) was divided by the body weight (g) of the mice to obtain a lung weight index. The results are shown in Fig. 13 and Table 11. It can be seen from Figure 13 that, compared to the sham operation group, the lung weight index of the mice was significantly increased after administration of bleomycin. Therefore, S19-S21 administration can significantly reduce the lung weight index of mice with fibrosis. In Fig. 13, "##" represents that P value is less than 0.01 when the lung weight index of the the administration groups were compared with that of the sham operation group; "*" represents that P value is less than 0.05 and "**" represents that P value is less than 0.01 when the lung weight index of the administration groups were compared with bleomycin group.

**Table 11 Effects of S119-21 on lung weight index in mice with pulmonary fibrosis**

| Groups | Lung weight index |
|---|---|
| sham operation group | 6.7875 |
| bleomycin group | 12.7375 |
| S19 administration group | 8.675 |
| S20 administration group | 10.0625 |
| S21 administration group | 11.2125 |

### Example 16 Determination of the effects of polypeptide S19-S21 on pathomorphology of bleomycin-induced pulmonary fibrosis

Pulmonary fibrosis models were made according to the method described in Example 2 and administered in groups according to Table 9. After 28 days, the right lower lobe of lung tissue of the experimental animals was taken, and fixed by 4% (v/v) paraformaldehyde and embedded in paraffin. Section was performed on the largest cross-section of the paraffin block in which the lung tissue embedded, and basic pathological changes was observed through HE staining. The results are shown in Fig. 14. Fig. 14 shows that the administration of polypeptides S19-S21 can reduce lung inflammation caused by bleomycin, and effectively ameliorate lung injury and restore normal lung structure.

The inflammatory grading was observed according to the results of HE staining, and the criteria were as follows (grade 0-5): grade "0": normal tissue; grade "1": extremely small inflammatory changes; grade "2": mild to moderate inflammatory changes without apparent destruction of lung tissue structure; grade "3": moderate inflammatory injury (thickening of alveolar diaphragm); grade "4": moderate to severe inflammatory injury with formation of tissue mass, or the normal structure of lung tissue was destroyed by localized pneumonia area; grade "5": severe inflammatory injury, severe destruction of the localized lung tissue structure, resulting in closure of lumen.

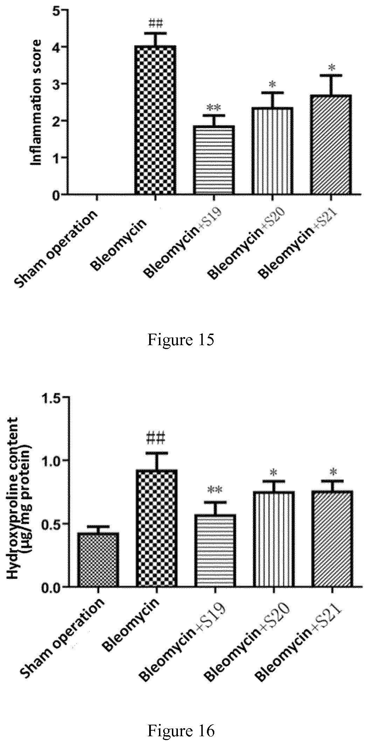

The results are shown in Figs. 15 and Table 12. The results showed that after administration of bleomycin, significant inflammation occurred in the lungs of mice compared to the sham-operated group. Administration of polypeptides S19-S21 even can significantly reduce lung inflammation caused by bleomycin. In Fig. 15, "##" represents that P value is less than 0.01 when inflammation score of the administration group was compared with that of the sham operation group; "*" represents that P value is less than 0.05 and "**" represents that P value is less than 0.01 when inflammation score of the administration group was compared with that of bleomycin group.

**Table 12 Inflammatory score of peptides S19-S21 on bleomycin-induced pulmonary fibrosis**

| Groups | Inflammatory scoring |
|---|---|
| sham operation group | 0 |
| bleomycin group | 4 |
| S19 administration group | 1.833333 |
| S20 administration group | 2.333333 |
| S21 administration group | 2.666667 |

### Example 17 Effects of polypeptides S19-S21 on hydroxyproline content in mice with pulmonary fibrosis were determined

Hydroxyproline accounts for 13.4% (w/w) of collagen, and accounts for extremely small amount of elastin, while it is absent in other proteins. Hence the collagen content was detected by hydroxyproline. Pulmonary fibrosis models were prepared according to the method described in Example 2 and administered in groups according to Table 3. The content of hydroxyproline in the all lobes of left lung of the animals was measured 28 days later, and the condition of pulmonary fibrosis was evaluated.

The specific method is as follows: the mice in the animal model prepared in Example 2 was feed in an SPF animal room and treated with polypeptide according to the dosing schedule of Table 3, and all the left lobes of lung of the experimental animal was taken on the 21st day after the animal model was constructed and the wet weight was recorded. Then 10% (w/w) tissue homogenate was prepared by ultrasonic homogenization using physiological saline. About 150 µL of homogenate supernatant was taken, and 500 µL of alkali hydrolysate was added (provided by Hydroxyproline alkaline hydrolysis kit of Nanjing Jiancheng Bioengineering Technology Co., Ltd.). After vortex mixing, the mixture was treated with alkaline hydrolysis for 40 min under conditions of 120 °C, 0.1Kpa. After adjusting the pH value, the volume was held constant, and the supernatant was taken after performing activated carbon treatment. The hydroxyproline was determined according to the chloramine-T method (the method of the present example refers to the instruction provided with the kit. Which is purchased from Nanjing Jiancheng Bioengineering Technology Co., Ltd.). The results are shown in Fig. 16 and Table 13. As can be seen from Fig. 16, compared with the sham operation group, the hydroxyproline content of the bleomycin group was significantly increased, indicating that the pathological changes of fibrosis were severe. Administration of polypeptides S19-S21 can significantly reduce the content of hydroxyproline in the lungs of mice with pulmonary fibrosis. In Fig. 16, "##" represents that P value is less than 0.01 when comparing the content of hydroxyproline in the administration group with that in the sham operation group; and "*" represents that P value is less than 0.05 and"**" represents that P value is less than 0.01 when comparing the content of hydroxyproline in the administration group with that in bleomycin group.

**Table 13 Effects of polypeptides S19-S21 on hydroxyproline content in mice with pulmonary fibrosis**

| groups | hydroxyproline content (µg/mg protein) |
|---|---|
| sham operation group | 0.42125 |
| bleomycin group | 0.915 |
| S19 administration group | 0.56375 |
| S20 administration group | 0.744286 |
| S21 administration group | 0.74875 |

### Example 18 Effects of polypeptides S19-S21 on pulmonary function in mice with pulmonary fibrosis were determined

Pulmonary function is a gold indicator for clinical detection of pulmonary fibrosis in patients. The decline of lung function is generally accompanied by an aggravation of fibrosis, while the improvement of lung function also generally represents the recovery of lung tissue structure. Pulmonary fibrosis models were prepared according to the method described in Example 2 and administered in groups according to Table 9. 28 days later, the mice were anesthetized with sodium pentobarbital (45 mg/kg, i.p.). Lung function was tested by the Flexivent Small-animal respiratory mechanics and the testing methods were TLC and SnapShots (Testing method refers to Lv X, Wang X, Li K, et al. Rupatadine Protects against Pulmonary Fibrosis by Attenuating PAF-Mediated Senescence in Rodents[J]. PloS one, 2013, 8(7): e68631*).*

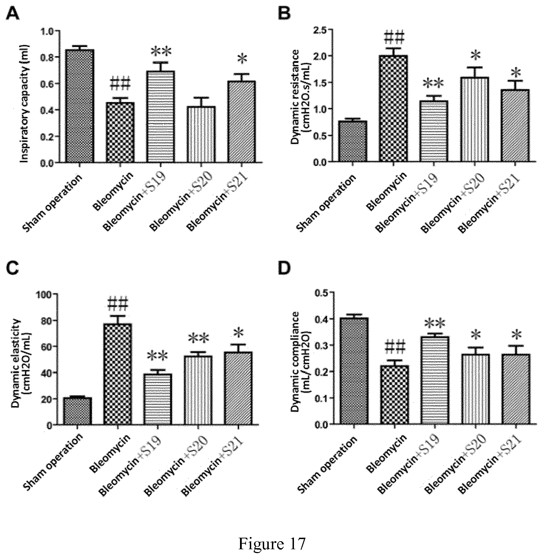

The results can be seen in Fig. 17 and Table 14, wherein "A" represents inspiratory capacity, "B" represents dynamic resistance, "C" represents dynamic elasticity and "D" represents dynamic compliance. Fig. 17 shows that, compared to the sham operation group, the inspiratory capacity of mice with bleomycin-induced pulmonary fibrosis was significantly reduced, the dynamic resistance and the dynamic elasticity of lungs increased, and the compliance was significantly reduced. Pulmonary function was significantly restored after treatment with peptides S19-S21. In Fig. 17, "##" represents that P value is less than 0.01 when comparing the effects of administration groups with that of the sham operation group; "*" represents that P value is less than 0.05 and "**" represents that P value is less than 0.01 when comparing the effects of administration groups with that of the bleomycin group.

**Table 14 Effects of polypeptides S19-S21 on pulmonary function in mice with pulmonary fibrosis**

| Groups | Inspiratory capacity (mL) | Dynamic resistance (cmH₂O.s/Ml) | Dynamic elasticity (cmH₂O./mL) | Dynamic compliance (mL/cmH₂O) |
|---|---|---|---|---|
| sham operation group | 0.852 | 0.758333 | 20.5 | 0.402 |
| bleomycin group | 0.452 | 1.996667 | 76.83333 | 0.22 |
| S19 administration group | 0.692 | 1.143333 | 38.66667 | 0.33 |
| S20 administration group | 0.422 | 1.588333 | 52.16667 | 0.264 |
| S21 administration group | 0.616 | 1.355 | 55.33333 | 0.264 |

The results of the above example show that the polypeptides of the present invention have a remarkable anti-pulmonary fibrosis effect and can be used as an active ingredient for the preparation of anti-pulmonary fibrosis drug.

Although specific embodiments of the present invention have been described above, it will be understood by those skilled in the art that these are merely illustrative and various changes or modifications may be made to these embodiments without departing from the principles and essence of the invention. Accordingly, these equivalent forms also fall within the scope of protection of the invention, which is defined by the appended claims.

## Claims

1. A polypeptide or derivatives thereof that specifically binds to TRB3, wherein the amino acid sequence of the polypeptide is represented by SEQ ID No. 12 in a Sequence List, or two or more amino acids in the amino sequence as represented by SEQ ID No. 12 in the Sequence List are replaced by unnatural amino acids having side chains that can be linked; the derivatives comprise a chimeric peptide formed by linking the polypeptide to a cell membrane penetrating peptide, a fusion peptide formed by the polypeptide and virus, the polypeptide that is methylated, the polypeptide that is glycosylated, and the polypeptide that is PEGylated.

2. The polypeptide or derivatives thereof that specifically binds to TRB3 according to claim 1, wherein the unnatural amino acid having side chains that can be linked is S-Pentenyl alanine; preferably the number of the replaced amino acids is two and the positions of the replaced amino acids are at position of i^{th} and (i+3)^{th} , or i^{th} and (i+4)^{th} respectively, wherein the "i" ranges from 1 to 11.

3. The polypeptide or derivatives thereof that specifically binds to TRB3 according to claim 2, wherein the polypeptide comprises amino acid sequence of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, and SEQ ID NO. 11 of the Sequence Listing.

4. The polypeptide or derivatives thereof that specifically binds to TRB3 according to any one of claims 1-3, wherein the cell membrane penetrating peptide is one or more peptides selected from TAT peptide of HIV-1 virus trans-activator transcription, the transcription factor of *Drosophila* antennal homeotic protein Antp peptide, Pep-1 peptide, Pep-2 peptide, MPG peptide and RGD peptide; preferably, the cell membrane penetrating peptide is linked to N terminal or C terminal of the polypeptide; more preferably:
Where the cell penetrating peptide is Pep-2 peptide, the derivative of the polypeptide comprises the amino acid sequence of SEQ ID No. 19;
Where the cell penetrating peptide is TAT peptide, the derivative of the polypeptide comprises the amino acid sequence of SEQ ID No. 20;
Where the cell penetrating peptide is Antp peptide, the derivative of the polypeptide comprises the amino acid sequence of SEQ ID No. 21.

5. An application of the polypeptide or derivatives thereof that specifically binds to TRB3 according to any one of claims 1-4 in preparation of drugs for treating and/or preventing pulmonary fibrosis.

6. The application of claim 5, wherein the pulmonary fibrosis is primary pulmonary fibrosis or secondary pulmonary fibrosis.

7. The application of claim 5, wherein the pulmonary fibrosis is induced by bleomycin.

8. A pharmaceutical composition having resistance to pulmonary fibrosis comprises the polypeptide or derivatives thereof that specifically binds to TRB3 according to any one of claims 1-4.

9. The pharmaceutical composition of claim 8, wherein it further comprises one or more pharmaceutical carriers.

10. The pharmaceutical composition of claim 8 or 9, wherein it comprises both of the polypeptide or derivatives thereof that specifically binds to TRB3 according to any one of claims 1-4 and compound having anti-pulmonary fibrosis activity as active ingredients.


SEQUENCE LISTING

<110>  Zhuowei Hu

<120>  Polypeptide, derivatives thereof, and application thereof in
       preparation of drugs having resistance to pulmonary fibrosis

<130>  P184115139EP

<140>  PCT/CN2017/074792
<141>  2017-02-24

<150>  CN 201610108248.9
<151>  2016-02-26

<160>  21

<170>  PatentIn version 3.5

<210>  1
<211>  15
<212>  PRT
<213>  Artificial sequence

<220>
<223>  S1


<220>
<221>  S-Pentenyl alanine
<222>  (1)..(1)

<220>
<221>  S-Pentenyl alanine
<222>  (5)..(5)

<400>  1

Xaa Leu Met Ala Xaa Phe Thr Cys Ala Lys Lys Leu Lys Lys Arg
1               5                   10                  15


<210>  2
<211>  15
<212>  PRT
<213>  Artificial sequence

<220>
<223>  S2


<220>
<221>  S-Pentenyl alanine
<222>  (2)..(2)

<220>
<221>  S-Pentenyl alanine
<222>  (6)..(6)

<400>  2

His Xaa Met Ala Cys Xaa Thr Cys Ala Lys Lys Leu Lys Lys Arg
1               5                   10                  15

<210>  3
<211>  15
<212>  PRT
<213>  Artificial sequence

<220>
<223>  S3



<220>
<221>  S-Pentenyl alanine
<222>  (3)..(3)

<220>
<221>  S-Pentenyl alanine
<222>  (7)..(7)

<400>  3

His Leu Xaa Ala Cys Phe Xaa Cys Ala Lys Lys Leu Lys Lys Arg
1               5                   10                  15


<210>  4
<211>  15
<212>  PRT
<213>  Artificial sequence

<220>
<223>  S4



<220>
<221>  S-Pentenyl alanine
<222>  (4)..(4)

<220>
<221>  S-Pentenyl alanine
<222>  (8)..(8)

<400>  4

His Leu Met Xaa Cys Phe Thr Xaa Ala Lys Lys Leu Lys Lys Arg
1               5                   10                  15


<210>  5
<211>  15
<212>  PRT
<213>  Artificial sequence

<220>
<223>  Xaa



<220>
<221>  S-Pentenyl alanine
<222>  (5)..(5)

<220>
<221>  S-Pentenyl alanine

<222>  (9)..(9)

<400>  5

His Leu Met Ala Xaa Phe Thr Cys Xaa Lys Lys Leu Lys Lys Arg
1               5                   10                  15


<210>  6
<211>  15
<212>  PRT
<213>  Artificial sequence

<220>
<223>  S6


<220>
<221>  S-Pentenyl alanine
<222>  (6)..(6)

<220>
<221>  S-Pentenyl alanine
<222>  (10)..(10)

<400>  6

His Leu Met Ala Cys Xaa Thr Cys Ala Xaa Lys Leu Lys Lys Arg
1               5                   10                  15


<210>  7
<211>  15
<212>  PRT
<213>  Artificial sequence

<220>
<223>  S7


<220>
<221>  S-Pentenyl alanine
<222>  (7)..(7)

<220>
<221>  S-Pentenyl alanine
<222>  (11)..(11)

<400>  7

His Leu Met Ala Cys Phe Xaa Cys Ala Lys Xaa Leu Lys Lys Arg
1               5                   10                  15


<210>  8
<211>  15
<212>  PRT
<213>  Artificial sequence

<220>
<223>  S8

<220>
<221>  S-Pentenyl alanine
<222>  (8)..(8)

<220>
<221>  S-Pentenyl alanine
<222>  (12)..(12)

<400>  8

His Leu Met Ala Cys Phe Thr Xaa Ala Lys Lys Xaa Lys Lys Arg
1               5                   10                  15


<210>  9
<211>  15
<212>  PRT
<213>  Artificial sequence

<220>
<223>  S9



<220>
<221>  S-Pentenyl alanine
<222>  (9)..(9)

<220>
<221>  S-Pentenyl alanine
<222>  (13)..(13)

<400>  9

His Leu Met Ala Cys Phe Thr Cys Xaa Lys Lys Leu Xaa Lys Arg
1               5                   10                  15


<210>  10
<211>  15
<212>  PRT
<213>  Artificial sequence

<220>
<223>  S10



<220>
<221>  S-Pentenyl alanine
<222>  (10)..(10)

<220>
<221>  S-Pentenyl alanine
<222>  (14)..(14)

<400>  10

His Leu Met Ala Cys Phe Thr Cys Ala Xaa Lys Leu Lys Xaa Arg
1               5                   10                  15


<210>  11
<211>  15
<212>  PRT

<213>  Artificial sequence

<220>
<223>  S11

<220>
<221>  S-Pentenyl alanine
<222>  (11)..(11)

<220>
<221>  S-Pentenyl alanine
<222>  (15)..(15)

<400>  11

His Leu Met Ala Cys Phe Thr Cys Ala Lys Xaa Leu Lys Lys Xaa
1               5                   10                  15

<210>  12
<211>  15
<212>  PRT
<213>  Artificial sequence

<220>
<223>  MR2 polypeptide

<400>  12

His Leu Met Ala Cys Phe Thr Cys Ala Lys Lys Leu Lys Lys Arg
1               5                   10                  15

<210>  13
<211>  11
<212>  PRT
<213>  Human immunodeficiency virus type 1

<220>
<221>  PEPTIDE
<222>  (1)..(11)
<223>  TAT peptide£¨S13£©

<400>  13

Tyr Gly Arg Lys Lys Arg Arg Gln Arg Arg Arg
1               5                   10

<210>  14
<211>  16
<212>  PRT
<213>  Drosophila melanogaster

<220>
<221>  PEPTIDE
<222>  (1)..(16)
<223>  Antp peptide£¨S14£©

<400>  14

Arg Gln Ile Lys Ile Trp Phe Gln Asn Arg Arg Met Lys Trp Lys Lys
1               5                   10                  15

<210>  15
<211>  21
<212>  PRT
<213>  Artificial sequence

<220>
<223>  Pep-1 peptideS15

<400>  15

Lys Glu Thr Trp Trp Glu Thr Trp Trp Thr Glu Trp Ser Gln Pro Lys
1               5                   10                  15

Lys Lys Arg Lys Val
            20

<210>  16
<211>  27
<212>  PRT
<213>  Human immunodeficiency virus

<220>
<221>  PEPTIDE
<222>  (1)..(27)
<223>  MPG peptide£¨S16£©

<400>  16

Gly Ala Leu Phe Leu Gly Phe Leu Gly Ala Ala Gly Ser Thr Met Gly
1               5                   10                  15

Ala Trp Ser Gln Pro Lys Ser Lys Arg Lys Val
            20                  25

<210>  17
<211>  3
<212>  PRT
<213>  Artificial sequence

<220>
<223>  RGD peptideS17

<400>  17

Arg Gly Asp
1

<210>  18
<211>  7
<212>  PRT
<213>  Artificial sequence

<220>
<223>  Pep2 peptideS18

<400>  18

His Leu Tyr Val Ser Pro Trp
1               5


<210>  19
<211>  24
<212>  PRT
<213>  Artificial sequence

<220>
<223>  chimeric polypeptide Pep2-MR2 S19

<400>  19

His Leu Tyr Val Ser Pro Trp Gly Gly His Leu Met Ala Cys Phe Thr
1               5                   10                  15


Cys Ala Lys Lys Leu Lys Lys Arg
            20


<210>  20
<211>  28
<212>  PRT
<213>  Artificial sequence

<220>
<223>  chimeric polypeptide TAT-MR2 S20

<400>  20

Tyr Gly Arg Lys Lys Arg Arg Gln Arg Arg Arg Gly Gly His Leu Met
1               5                   10                  15


Ala Cys Phe Thr Cys Ala Lys Lys Leu Lys Lys Arg
            20                  25


<210>  21
<211>  33
<212>  PRT
<213>  Artificial sequence

<220>
<223>  chimeric polypeptide Antp-MR2 S21

<400>  21

Arg Gln Ile Lys Ile Trp Phe Gln Asn Arg Arg Met Lys Trp Lys Lys
1               5                   10                  15


Gly Gly His Leu Met Ala Cys Phe Thr Cys Ala Lys Lys Leu Lys Lys
            20                  25                  30

Arg